# EUROPÄISCHE PATENTSCHRIFT

(11) **EP 0 574 696 B1**
(45) Veröffentlichungstag und Bekanntmachung des Hinweises auf die Patenterteilung: **17.07.1996**
(21) Anmeldenummer: 93107542.8
(22) Anmeldetag: 10.05.1993
(51) Int. Cl.: A61K 7/135

(54) **Verfahren zur Herstellung von pulverförmigen Mitteln zum Blondieren von menschlichen Haaren**
Manufacturing process of powdered human hair bleaching composition
Procédé de préparation de compositions poudreuses de décoloration des cheveux humains

(30) Priorität: 30.05.1992 DE 4217920; 21.08.1992 DE 4227674
(43) Veröffentlichungstag der Anmeldung: 22.12.1993
(73) Patentinhaber: GOLDWELL GmbH, D-64297 Darmstadt (DE)
(72) Erfinder: Lorenz, Heribert, W-6101 Gross-Bieberau (DE); Hirschfeld, Peter, W-7800 Freiburg (DE)

(56) Entgegenhaltungen:
- EP-A- 0 452 145
- DE-A- 2 023 922
- US-A- 3 089 824

## Beschreibung

Die vorliegende Erfindung betrifft ein neues Verfahren zur Herstellung von pulverförmigen Mitteln zum Blondieren von menschlichen Haaren mit verbesserten Gebrauchseigenschaften.

Herkömmliche Mittel zum Blondieren bzw. Bleichen von menschlichen Haaren bestehen aus mindestens einem festen Peroxid, insbesondere einem Persulfat, und einem pulverförmigen Trägermaterial. Dieses Pulver wird bei Gebrauch mit einer 6- bis 12-prozentigen Wasserstoffperoxid-Lösung angerührt und auf das Haar aufgebracht. Beispiele für derartige Zusammensetzungen finden sich in der einschlägigen Fachliteratur, beispielsweise bei K.Schrader, "Grundlagen und Rezepturen der Kosmetika", 2. Auflage (1989, Hüthig Buchverlag), S.815 bis 823.

Die Gebrauchseigenschaften dieser Blondierpulver sind jedoch bisher nicht befriedigend. Sie stauben nicht nur bei der Anwendung, sondern sind auch bei der Dosierung nicht exakt handhabbar, wodurch das erwünschte Bleichergebnis beeinträchtigt werden kann.

Es wurde nunmehr gefunden, daß ein pulverförmiges Mittel zum Blondieren von menschlichen Haaren, das die erwähnten Nachteile nicht aufweist, insbesondere nicht mehr staubt, erhalten werden kann, wenn man das pulverförmige Ausgangsmaterial, das mindestens ein Peroxid, vorzugsweise ein Persulfat enthält, durch Zusatz eines Binde- bzw. Verdickungsmittels agglomeriert, wobei in einer ersten Stufe das pulverförmige Ausgangsmaterial mit einem Bindemittel behandelt und in einer zweiten Stufe ein weiteres Binde- und Verdickungsmittel dem Gemisch zugesetzt und anschließend das erhaltene Agglomerat getrocknet wird.

Auf diese Weise wird ein stabiles staubfreies Produkt erhalten, das sich problemlos vor der Anwendung auf dem Haar mit wäßrigen Lösungen, beispielsweise der üblichen Wasserstoffperoxid-Lösung, homogen vermischen und auftragen läßt.

Aus der DE-OS 2 023 922 sind bereits granulierte Haarbleichmittel bekannt, die aus Persalzen und wasserlöslichen Bindemitteln, insbesondere Polyvinylpyrrolidon, hergestellt worden sind. Diese Granulate vermögen jedoch die oben angesprochenen Probleme deshalb nicht zu lösen, weil sie relativ große Teilchengrößen im Millimeterbereich aufweisen und beim Anrühren mit wäßrigem Wasserstoffperoxid nur schwer in Lösung gehen.

Gleiches gilt für die aus der DE-OS 40 26 235 bekannten Granulate aus Persulfat, die praktisch den aus der vorgenannten DE-OS 2 023 922 bekannten Produkten entsprechen.

Wichtig bei dem zweistufigen Beschichtungs- bzw. Agglomerierungsverfahren nach der Erfindung ist, daß in der zweiten Phase ein Bindemittel eingesetzt wird, das gleichzeitig eine verdickende Wirkung aufweist.

Dabei liegt das Gewichtsverhältnis zwischen dem in der ersten Stufe eingesetzten Bindemittel und dem in der zweiten Stufe zugesetzten Binde- bzw. Verdickungsmittel bei etwa 1 : 2 bis 1 : 50, vorzugsweise 1 : 3 bis 1 : 10, insbesondere 1 : 4 bis 1 : 6. Die Teilchengröße des erfindungsgemäß hergestellten Blondiermittel-Agglomerats liegt dabei zwischen 50 und 1000 µm, vorzugsweise 200 bis 500 µm, die sich, wie bereits ausgeführt, ausgezeichnet eignen, um mit wäßrigen Lösungen zubereitet zu werden.

Geeignete Bindemittel zur Durchführung des erfindungsgemäßen Verfahrens sind im Prinzip alle wasserlöslichen, für diesen Zweck bekannten natürlichen und synthetischen Polymeren.

Als solche sind insbesondere die verschiedenen Cellulose-Derivate, beispielsweise Alkylcellulosen wie Methylcellulose und Ethylcellulose, Hydroxyalkylcellulosen wie Hydroxyethylcellulose, Hydroxypropylcellulose und Methylhydroxypropylcellulose sowie auch Carboxymethylcellulose genannt; weiterhin Stärke und Stärkederivate; Gelatine, natürliche Gummen wie Guar-Gum oder Xanthan-Gum oder auch Alkalialginate und Alginsäureester, beispielsweise Propylenglykolalginat, sowie Polyethylenglykole mit einem Molgewicht ab 10 000, sowie Dextrine, insbesondere Maltodextrin.

Geeignete synthetische Polymere sind beispielsweise Polyvinylpyrrolidon, Polyacrylamid, Alkalisalze der Polyacrylsäure, etc.

Die obengenannten Bindemittel weisen gleichzeitig eine verdickende Wirkung auf und sind daher geeignet, in beiden Stufen des erfindungsgemäßen Verfahrens zum Einsatz zu gelangen.

Geeignete Bindemittel, die in der ersten Verfahrensphase zugesetzt werden, jedoch keine oder nur eine geringe verdickende Wirkung aufweisen und deshalb zum Zusatz in der zweiten Verfahrensphase nicht oder nur weniger geeignet sind, sind beispielsweise Acrylestercopolymerisate mit Acrylsäure und/oder Dialkylaminoalkyl(meth)acrylaten, beispielsweise Diethyl- und Dimethylaminoethylmethacrylat, die gegebenenfalls auch quaternisiert sein können, neutralisierte Copolymerisate aus Methacrylsäure und Methylmethacrylat sowie weitere bekannte wasserlösliche Copolymere.

Die Gesamtmenge des aufgebrachten Bindemittels in beiden Herstellungsstufen liegt bei etwa 0,5 bis etwa 20 Gew.-%, insbesondere 2 bis 15 Gew.-%, vorzugsweise 5 bis etwa 10 Gew.-%, der Gesamtzusammensetzung des agglomerierten Blondierpulvers.

Der Feuchtigkeitsgehalt der erfindungsgemäßen Agglomerate, der sich auf die Lagerstabilität der enthaltenen Perverbindungen nachteilig auswirken kann, kann gemäß einer bevorzugten Ausführungsform der Erfindung noch herabgesetzt werden, wenn eine Vor-Agglomerierungsstufe durchgeführt wird.

Dabei wird zunächst das pulverförmige, den Hauptanteil der Perverbindungen und mindestens ein Alkalisierungsmittel enthaltende Ausgangsmaterial mit einem in einer wäßrig/alkoholischen Lösung gelösten Bindemittel behandelt, anschließend vorgetrocknet, dann werden die restlichen Bestandteile eingebracht und mit einem Bindemittel behandelt. Anschließend erfolgt, wie beschrieben, die weitere Aufbringung eines weiteren Binde- und Verdickungsmittels und das Trocknen des Agglomerats bei erhöhter Temperatur.

Als wäßrig/alkoholische Lösung für den Vor-Agglomierungsprozeß wird ein Gemisch aus Wasser und einem niedrigen Alkohol, insbesondere Ethyl-, n-Propyl- und/oder Isopropylalkohol, vorzugsweise im Gewichtsverhältnis von 0,5 : 1 bis 3 : 1, eingesetzt.

Als Bindemittel wird in dieser Vor-Agglomerisierungsstufe bevorzugt ein Cellulosederivat wie Hydroxypropylmethylcellulose verwendet. Das mit den Perverbindungen eingesetzte Alkalisierungsmittel ist vorzugsweise Natriumsilikat wie Natriummetasilikat.

Unter dem Begriff "Hauptanteil der Perverbindungen" ist zu verstehen, daß dies mehr als 50 Gew.-% der im Gesamtprodukt vorhandenen Peroxide und Persulfate, Perborate, etc. sein sollen, vorzugsweise mehr als 70, insbesondere 100 % derselben.

Verbessert werden können die Gebrauchseigenschaften des erfindungsgemäß hergestellten Blondierpulvers noch durch die Mitverwendung geringer Mengen von Tensiden, insbesondere anionischen und nichtionischen Tensiden.

Als solche sind insbesondere die bekannten langkettigen Alkyl- und Alkylethersulfate, Fettsäuretauride und Olefinsulfonate sowie C₁₂-C₁₈-Fettalkohol-Polyglykolether, beispielsweise mit 3 bis 15 Ethylenoxid-Einheiten pro Mol, und Alkylphenolpolyglykolether, beispielsweise Nonylphenolpolyglykolether mit etwa 4 bis etwa 10 Ethylenoxid-Einheiten pro Mol, geeignet.

Deren Anteil liegt vorzugsweise bei etwa 0,1 bis etwa 5 Gew.-%, vorzugsweise 0,15 bis 2,5 Gew.-%, insbesondere bis etwa 1 Gew.-%, berechnet auf die Gesamtzusammensetzung des erfindungsgemäß hergestellten Blondierpulvers.

Wie bereits angedeutet, enthalten die Blondiermittel die in solchen Mitteln bekannten und üblichen Bestandteile, es wird hierzu zur Vermeidung von Wiederholungen auf Schrader, l.c., verwiesen.

Geeignete Peroxide sind hierbei insbesondere Alkalipersulfate wie Kalium- und Natriumpersulfat sowie insbesondere auch Ammoniumpersulfat; es können auch Magnesiumperoxid, Harnstoffperoxid, Melaminperoxid etc. sowie insbesondere auch Gemische der genannten Peroxide mit den genannten Persulfaten verwendet werden.

Die Herstellung nach der Erfindung erfolgt im Prinzip auf folgende Weise:
Zunächst wird das pulverförmige Mittel, vorzugsweise in einer Wirbelschichtsprühanlage, mit einer Lösung des Bindemittels, die vorzugsweise alkalisch eingestellt ist, besprüht. Hierzu wird zweckmäßigerweise eine Wirbelschicht-Topsprayanlage mit Unterdruckbetrieb verwendet.

Das Sprühen erfolgt vorzugsweise bei einer Temperatur zwischen etwa 25 und etwa 50 °C, vorzugsweise 30 bis etwa 40 °C.

Nach dem vollständigen Aufbringen der Bindemittel-Lösung wird anschließend, vor dem Trocknen, der Rest des Bindemittels, der gleichzeitig als Verdickungsmittel wirkt, angesaugt und nach kurzem Verwirbeln das erhaltene Produkt getrocknet.

Geeignete Wirbelschichtverfahren sind aus der Literatur hinreichend bekannt, beispielsweise beschreiben Kala, Dittken und Moldenhauer in Pharmazie Nr. 11/1971 ein solches Verfahren.

Ein entsprechendes Verfahren ist auch als "Wurster"-Verfahren bekannt und im Journal of the American Pharmaceutical Association, Vol. 48 (1959), S.451 von D.Wurster beschrieben.

Eine weitere Beschreibung von A. M. Mehta, M.J.Valazza und St.E.Abele findet sich in Pharmaceutical Technology, April 1986, "Evaluation of fluid-bed processes for enteric coating systems".

Im folgenden wird anhand eines Beispiels das erfindungsgemäße Verfahren zur Herstellung eines staubfreien Blondierungspulvers erläutert:
Eine Mischung aus
- 5,30 (kg): Pyrogenem Siliciumdioxid
- 0,85: Hydroxyethylcellulose
- 2,30: Natriumcarboxymethylcellulose
- 0,25: EDTA, Trinatriumsalz
- 2,60: Harnstoff
- 0,75: Natriumstearat
- 0,85: Natriumcarbonat
- 2,85: Stärkepulver
- 0,50: Kokosfettsäuretaurid, Natriumsalz (50%ig)
- 20,00: Natriummetasilikat
- 3,60: Magnesiumperoxid
- 30,00: Ammoniumpersulfat
- 30,00: Kaliumpersulfat
- 0,15: Farbstoff
wird in eine Wirbelschicht-Topsprayanlage eingebracht und mit 38,5 kg einer Lösung aus
- 0,53 kg: NaOH
- 7,89 kg: Methacrylsäure/Methylmethacrylat-Copolymerisat (Eudragit^{R} L30D)-Dispersion (30 % Feststoff-Gehalt)
- in 30,08 kg: Wasser
bei 25 bis 35 °C besprüht.

Nach beendetem Sprühvorgang werden nach 35 Minuten 5,2 kg eines Gemisches aus 1,2 kg Hydroxyethylcellulose und 4,0 kg Natriumcarboxymethylcellulose innerhalb 5 Minuten eingezogen und anschließend das erhaltene Agglomerat sofort bei etwa 35° - 45 °C getrocknet.

Das erhaltene Produkt ist absolut staubfrei, besitzt ausgezeichnete Rieselfähigkeit, klebt nicht und läßt sich mit 9%iger Wasserstoffperoxid-Lösung zu einem gut auf das Haar aufzutragenden Blondiermittel anrühren.

Jeweils weniger als 1 % der Teilchen weisen einen Durchmesser unterhalb 50 µm und oberhalb 900 µm auf.

Ein Produkt mit annähernd gleich guten Eigenschaften wird erhalten, wenn in der ersten Agglomerierungsstufe eine Mischung aus 8 Gew.-% Polyethylenglykol 10 000/Polyethylenglykol 20 000 im Verhältnis 1 : 1 in 92 Gew.-% eines 50 : 50-Gemisches aus Isopropylalkohol/Wasser aufgesprüht und anschließend ein Hydroxyethylcellulose/Carboxymethylcellulose-Verdickungsmittel-Gemisch entsprechend dem obenbeschriebenen Beispiel zugesetzt wird.

Ein weiteres Verfahren mit Vor-Agglomerierung wird folgendermaßen durchgeführt:

### Zusammensetzung des Blondiermittels vor der Agglomerisation:

| | | |
|---|---|---|
| A: | Magnesiumperoxid (25%ig) | 180 g |
| | Ammoniumpersulfat | 1500 g |
| | Kaliumpersulfat | 1500 g |
| | Natriummetasilikat | 900 g |
| B: | Pyrogene Kieselsäure | 250 g |
| | Natriumcocosfettsäuretaurid (50%ig) | 25 g |
| | Hydroxyethylcellulose | 32 g |
| | Carboxymethylcellulose | 110 g |
| | Komplexbildner (EDTA-Na) | 10 g |
| | Eiweißhydrolysat | 25 g |
| | Natriumstearat | 35 g |
| | Harnstoff | 125 g |
| | Stärke | 150 g |
| | Natriumcarbonat | 40 g |

Die in der vorstehenden Rezeptur mit A bezeichneten Bestandteile (4080 g) wurden in eine Wirbelschicht-Topspray-Anlage mit Unterdruckbetrieb eingebracht und mit einer Lösung von 260 g Hydroxypropylmethylcellulose in einem Gemisch aus 1730 g Wasser und 1200 g Isopropylalkohol bei etwa 35 bis etwa 60 °C 45 bis 60 Minuten besprüht und anschließend 15 Minuten bei etwa 40 bis etwa 60 °C getrocknet, worauf ein Wassergehalt von lediglich etwa 4 % erreicht wurde.

Anschließend wurden die Bestandteile der Zusammensetzung B (802 g) zugemischt und, entsprechend der Lehre des Hauptpatents, eine Mischung aus
- 63 g: NaOH,
- 195 g: Methacrylsäure/Methylmethacrylat-Copolymerisat-Dispersion (Eudragit^{R} L30D) und
- 2700 g: Wasser,
bei etwa 25 bis etwa 35 °C während etwa 30 bis 45 Minuten aufgesprüht.

Nach beendetem Sprühvorgang werden 650 g eines Gemisches aus 150 g Hydroxyethylcellulose und 500 g Natriumcarboxymethylcellulose innerhalb 5 bis 15 Minuten eingezogen und anschließend das erhaltene Agglomerat sofort bei etwa 35° bis etwa 55 °C getrocknet.

Das erhaltene Produkt ist absolut staubfrei, besitzt ausgezeichnete Rieselfähigkeit, klebt nicht und läßt sich mit 9%iger Wasserstoffperoxid-Lösung zu einem gut auf das Haar aufzutragenden Blondiermittel anrühren.

Jeweils weniger als 1 % der Teilchen weisen einen Durchmesser unterhalb 50 µm und oberhalb 900 µm auf.

Der Wassergehalt liegt bei maximal 4 Gew.-%.

Das in der Vor-Agglomerierung eingesetzte Bindemittel ist vorzugsweise, wie das in der letzten Stufe eingesetzte Produkt, ein solches mit gleichzeitig verdickender Wirkung.

## Patentansprüche

1. Verfahren zur Herstellung von nichtstaubenden pulverförmigen Mitteln zum Blondieren von menschlichen Haaren, wobei das pulverförmige Ausgangsmaterial durch Zusatz eines Binde- bzw. Verdickungsmittels agglomeriert wird, dadurch gekennzeichnet, daß das mindestens ein Peroxid enthaltende pulverförmige Ausgangsmaterial in einer ersten Stufe mit einem Bindemittel behandelt und in einer zweiten Stufe ein weiteres Binde- und Verdickungsmittel dem Gemisch zugesetzt und anschließend das erhaltene Agglomerat getrocknet wird.

2. Verfahren nach Anspruch 1, dadurch gekennzeichnet, daß das Gewichtsverhältnis des in der ersten Stufe eingesetzten Bindemittels zu dem in der zweiten Stufe zugesetzten Binde- und Verdickungsmittel bei 1 : 2 bis 1 : 50 liegt.

3. Verfahren nach Anspruch 2, dadurch gekennzeichnet, daß das Verhältnis des in der ersten Stufe eingesetzten Bindemittels zu dem in der zweiten Stufe zugesetzten Binde- und Verdickungsmittel bei 1 : 3 bis 1 : 10 liegt.

4. Verfahren nach Anspruch 3, dadurch gekennzeichnet, daß das Gewichtsverhältnis des in der ersten Stufe eingesetzten Bindemittels zu dem in der zweiten Stufe zugesetzten Binde- und Verdickungsmittel bei etwa 1 : 4 bis 1 : 6 liegt.

5. Verfahren nach einem der vorhergehenden Ansprüche, dadurch gekennzeichnet, daß das in der ersten Stufe eingesetzte Bindemittel und das in der zweiten Stufe zugesetzte Binde- und Verdickungsmittel identisch sind.

6. Verfahren nach einem der Ansprüche 1 bis 4, dadurch gekennzeichnet, daß es sich bei dem in der ersten Stufe eingesetzten Bindemittel und dem in der zweiten Stufe zugesetzten Binde- und Verdickungsmittel um verschiedene Substanzen handelt.

7. Verfahren nach Anspruch 6, dadurch gekennzeichnet, daß es sich bei dem in der ersten Stufe eingesetzten Bindemittel um ein neutralisiertes Copolymerisat aus Methacrylsäure und Methylmethacrylat handelt.

8. Verfahren nach einem der vorhergehenden Ansprüche, dadurch gekennzeichnet, daß es sich bei dem in der zweiten Stufe zugesetzten Binde- und Verdickungsmittel um ein oder mehrere wasserlösliche Cellulosederivate handelt.

9. Verfahren nach einem oder mehreren der vorhergehenden Ansprüche, dadurch gekennzeichnet, daß in einer Vor-Agglomerisationsstufe ein pulverförmiges, den Hauptanteil der Perverbindungen und mindestens ein Alkalisierungsmittel enthaltendes Gemisch mit mindestens einem in einer wäßrig/alkoholischen Lösung gelösten Bindemittel behandelt und anschließend vorgetrocknet wird.

10. Verfahren nach Anspruch 9, dadurch gekennzeichnet, daß als Bindemittel ein Cellulosederivat eingesetzt wird.

11. Verfahren nach Anspruch 10, dadurch gekennzeichnet, daß als Bindemittel Hydroxyethylcellulose, Hydroxypropylcellulose und/oder Hydroxypropylmethylcellulose eingesetzt wird.

## Claims

1. Process for the production of non-dusting pulverulent compositions for bleaching human hair, wherein the powdery basic material is agglomerated by the addition of a binding or thickening agent, characterized in that the powdery basic material contains at least one peroxide and is treated with a binding agent in the first stage, and in a second stage a further binding and thickening agent is added to the mixture, whereupon the agglomerate obtained is dried.

2. Process according to claim 1, characterized in that the weight proportion of the binding agent used in the first stage to the binding and thickening agent used in the second stage is 1 : 2 to 1 : 50.

3. Process according to claim 2, characterized in that the proportion of the binding agent used in the first stage to the binding and thickening agent used in the second stage is 1 : 3 to 1 : 10.

4. Process according to claim 3, characterized in that the weight proportion of the binding agent used in the first stage to the binding and thickening agent used in the second stage is about 1 : 4 to 1 : 6.

5. Process according to one of the preceding claims, characterized in that the binding agent used in the first stage and the binding and thickening agent used in the second stage are identical.

6. Process according to one of claims 1 to 4, characterized in that the binding agend used in the first stage and the binding and thickening agent added in the second stage are different substances.

7. Process according to claim 6, characterized in that the binding agend used in the first stage is a neutralized copolymer of methacrylic acid and methyl methacrylate.

8. Process according to one of the preceding claims, characterized in that the binding and thickening agent added in the second stage comprises one or several water-soluble cellulose derivatives.

9. Process according to one or more of the preceding claims, characterized in that a pulverulent mixture comprising the dominant proportion of the per-compounds and at least one alkalizing substance is treated with at least one thickening agent dissolved in an aqueous/alcoholic solution in a pre-agglomerating step whereupon the product is pre-dried.

10. Process according to claim 9, characterized in that the binding agent used in the pre-agglomerating step is a cellulose derivative.

11. Process according to claim 10, characterized in that hydroxyethyl cellulose, hydroxypropyl cellulose and (or) hydroxypropyl methyl cellulose is used as a binding agent.

## Revendications

1. Procédé pour la préparation de produits pulvérulents ne dégageant pas de poussière pour blondir les cheveux humains, dans lequel le produit de départ pulvérulent est aggloméré par addition d'un liant ou d'un agent épaississant, caractérisé en ce que le produit de départ pulvérulent qui contient au moins un peroxyde est traité par un liant au cours d'une première étape, et en ce qu'au cours d'une deuxième étape un autre agent liant - épaississant est ajouté au mélange, l'agglomérat obtenu étant ensuite séché.

2. Procédé selon la revendication 1, caractérisé en ce que le rapport du poids de liant ajouté au cours de la première étape au poids d'agent liant - épaississant ajouté au cours de la deuxième étape se situe entre 1/2 et 1/50.

3. Procédé selon la revendication 2, caractérisé en ce que le rapport du poids de liant ajouté au cours de la première étape au poids d'agent liant - épaississant ajouté au cours de la deuxième étape se situe entre 1/3 et 1/10.

4. Procédé selon la revendication 3, caractérisé en ce que le rapport du poids de liant ajouté au cours de la première étape au poids d'agent liant - épaississant ajouté au cours de la deuxième étape se situe entre environ 1/4 et 1/6.

5. Procédé selon l'une des revendications précédentes, caractérisé en ce que le liant ajouté au cours de la première étape est identique à l'agent liant - épaississant ajouté au cours de la deuxième étape.

6. Procédé selon l'une des revendications 1 à 4, caractérisé en ce que le liant ajouté au cours de la première étape et l'agent liant - épaississant ajouté au cours de la deuxième étape sont des substances différentes.

7. Procédé selon la revendication 6, caractérisé en ce que le liant ajouté au cours de la première étape est un copolymérisat neutralisé d'acide méthacrylique et de méthacrylate de méthyle.

8. Procédé selon l'une des revendications précédentes, caractérisé en ce que l'agent liant - épaississant ajouté au cours de la deuxième étape est constitué par un ou plusieurs dérivés de cellulose solubles dans l'eau.

9. Procédé selon l'une ou plusieurs des revendications précédentes, caractérisé en ce que, au cours d'une étape préliminaire d'agglomération, un mélange pulvérulent contenant la plus grande partie du composé peroxydé et au moins un agent alcalinisant est traité par au moins un liant dissous dans une solution aqueuse/-alcoolique, puis séché.

10. Procédé selon la revendication 9, caractérisé en ce que l'on ajoute un dérivé de cellulose en tant que liant.

11. Procédé selon la revendication 10, caractérisé en ce qu'on ajoute en tant que liant, une hydroxyéthylcellulose, une hydroxypropylcellulose et/ou une hydroxypropylméthylcellulose.
